# EUROPÄISCHE PATENTSCHRIFT

(11) **EP 1 200 436 B1**
(45) Veröffentlichungstag und Bekanntmachung des Hinweises auf die Patenterteilung: **29.10.2008**
(21) Anmeldenummer: 00949322.2
(22) Anmeldetag: 12.07.2000
(51) Int. Cl.: C07D 475/04

(54) **VERFAHREN ZUR HERSTELLUNG REINER STEREOISOMEREN VON TETRAHYDROFOLSÄUREESTERSALZEN UND TETRAHYDROFOLSÄURE DURCH FRAKTIONIERTE KRISTALLISATION VON TETRAHYDROFOLSÄUREESTERSALZEN**
METHOD FOR MAKING PURE STEREOISOMERS OF TETRAHYDROFOLIC ACID ESTER SALTS AND TETRAHYDROFOLIC ACID BY FRACTIONATED CRYSTALLISATION OF TETRAHYDROFOLIC ACID SALTS
PROCEDE DE FABRICATION DE STEREOISOMERES PURS DE SELS D'ESTERS D'ACIDE TETRAHYDROFOLIQUE ET D'ACIDE TETRAHYDROFOLIQUE PAR CRISTALLISATION FRACTIONNEE DE SELS D'ESTERS D'ACIDE TETRAHYDROFOLIQUE

(30) Priorität: 14.07.1999 CH 130099
(43) Veröffentlichungstag der Anmeldung: 02.05.2002
(73) Patentinhaber: Merck Eprova AG, 8200 Schaffhausen (CH)
(72) Erfinder: MÜLLER, Hans, Rudolf, CH-8200 Schaffhausen (CH); MOSER, Rudolf, CH-8200 Schaffhausen (CH); GROEHN, Viola, CH-8212 Neuhausen am Rheinfall (CH)
(86) Internationale Anmeldenummer: PCT/EP2000/006647
(87) Internationale Veröffentlichungsnummer: WO 2001/004121

(56) Entgegenhaltungen:
- EP-A- 0 348 641
- EP-A- 0 495 204
- EP-A- 0 537 492
- EP-A- 0 682 026

## Beschreibung

Die vorliegende Erfindung betrifft ein Verfahren zur Herstellung und Anreicherung von (6S,αS)- oder (6S,αR)-Tetrahydrofolsäureestersalzen und -Tetrahydrofolsäure, indem man in einem organischen Lösungsmittel äquimolare oder angereicherte Mischungen von Diastereomeren von Additionssalzen von Tetrahydrofolsäureestern mit aromatischen Sulfonsäuren herstellt oder löst, anschliessend wenigstens einmal kristallisiert, und diese gegebenenfalls zu (6S,αS)-beziehungsweise (6S,αR)-Tetrahydrofolsäure hydrolysiert und diese als frei Säure kristallisiert oder in Form ihrer Salze isoliert. Aus den Mutterlaugen können die Additionssalze der (6R,αS)- beziehungsweise (6R,αR)-Tetrahydrofolsäureester mit den entsprechenden Sulfonsäuren isoliert und durch Hydrolyse die entsprechenden Tetrahydrofolsäuren oder deren Salze erhalten werden.

Folsäure entspricht der Formel I, 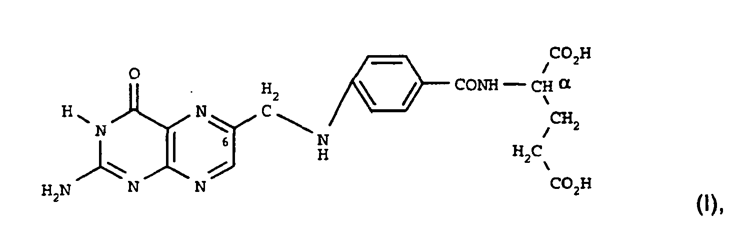 wobei das asymmetrische α-C-Atom im Glutaminsäurerest in der S-Konfiguration (αS) oder
in der R-Konfiguration (αR) vorliegen kann. Die Enantiomeren der Folsäure werden nachfolgend als (αS)-Folsäure und (αR)-Folsäure bezeichnet. Das Gleiche gilt für die Folsäureester und ihre Derivate. Sie werden als (αS)-Folsäureester und (αR)-Folsäureester bezeichnet. Die natürlich vorkommende Folsäure entspricht der (αS)-Folsäure.

Tetrahydrofolsäure entspricht der Formel II, 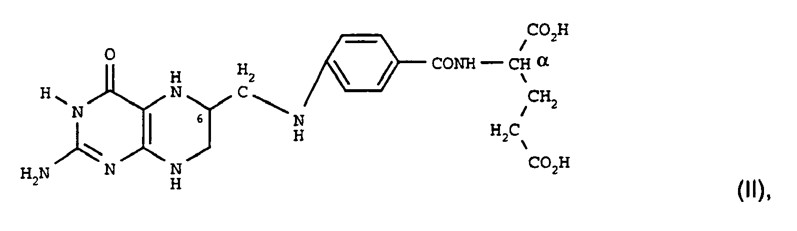 wobei das asymmetrische α-C-Atom im Glutaminsäurerest in der S-Konfiguration (αS) oder in der R-Konfiguration (αR) vorliegen kann und das asymmetrische C-Atom 6 im Tetrahydropterinrest in der S-Konfiguration (6S) oder R-Konfiguration (6R) vorliegen kann. Die Diastereomeren der Tetrahydrofolsäure werden nachfolgend als (6S,αS)-, (6S,αR)-, (6R,αS)- und (6R,αR)-Tetrahydrofolsäure bezeichnet. Das Gleiche gilt für die Tetrahydrofolsäureester und ihre Derivate. Sie werden als (6S,αS)-, (6S,αR)-, (6R,αS)- und (6R,αR)-Tetrahydrofolsäureester bezeichnet. Die natürlich vorkommende Tetrahydrofolsäure entspricht der (6S,αS)-Tetrahydrofolsäure.

Im Folgenden beinhaltet die Bezeichnung Folsäure, Folsäureester und Folsäureestersalze, falls nicht anderweitig bezeichnet, immer die beiden Enantiomeren (αS) und (αR) und die Bezeichnung Tetrahydrofolsäure, Tetrahydrofolsäureester und Tetrahydrofolsäureestersalze alle möglichen Diastereomeren.

Tetrahydrofolsäure hat in Form von 5-Formyl- oder 5-Methylderivaten und ihren physiologisch verträglichen Salzen eine breite therapeutische Anwendung gefunden. Es ist seit langem bekannt, dass die biologische Aktivität der natürlich vorkommenden Diastereomeren der reduzierten Folate, z. B. der (6S,αS)-Tetrahydrofolsäure, bei weitem am Stärksten ist. Es ist daher zweckmässig, therapeutische Präparate bereitzustellen, in dem nur die aktivste Form enthalten oder diese zumindest hoch angereichert ist.

Tetrahydrofolsäure wird industriell im allgemeinen durch heterogene Hydrierung der beiden Imingruppen im Pteringerüst von (αS)-Folsäure hergestellt, wobei man üblicherweise ein äquimolares Gemisch aus (6S,αS)- und (6R,αS)-Tetrahydrofolsäure erhält. Das äquimolare Gemisch kann für pharmazeutische Zubereitungen verwendet werden. Man kann aber auch zuvor das gewünschte (6S,αS)-Diastereomere der Tetrahydrofolsäure durch fraktionierte Kristallisation anreichern oder in reiner Form gewinnen, wofür verschiedene Verfahren bekannt sind, siehe zum Beispiel EP-0 495 204.

Das in der EP-0 495 204 beschriebene Verfahren verwendet die äquimolaren Mischungen von (6S,αS)- und (6R,αS)-Diastereomeren der Tetrahydrofolsäuresulfonsäuresalze, die in Wasser gelöst und dann kristallisiert werden. Dieses Verfahren führt zur Anreicherung des gewünschten (6S,αS)-Diastereomeren, wobei man bereits im ersten Kristallisationsschritt sehr hohe Anreicherungen erzielen (bis etwa 95%) und durch eine weitere fraktionierte Kristallisation reine (6S,αS)-Tetrahydrofolsäure erhalten kann. Dieses Verfahren kann unter anderem aus ökonomischer Sicht insofern nicht überzeugen, als die zur Salzbildung verwendeten Sulfonsäuren nur mit hohem Aufwand aus wässrigen Mutterlaugen abtrennbar sind, und daher die Entsorgung grosser Volumina sulfonsäurehaltiger Mutterlaugen vorgenommen werden muss, was wirtschaftlich ungünstig ist.

In der EP-0 682 026 wird die Herstellung von stabiler kristalliner (6S,αS)- und (6R,αS)-Tetrahydrofolsäure durch Kristallisation aus einem wässrigen Medium bei bestimmten pH-Werten beschrieben. Die Anreicherungen bei den fraktionierten Kristallisationen sind jedoch so niedrig, dass mehrere Schritte für eine Anreicherung des gewünschten Diastereomeren auf über 99.5% nötig sind. Damit verbunden sind grosse Substanzverluste und die Gefahr der Bildung chemischer Abbauprodukte. Speziell aufwändig nach diesem Verfahren ist die Anreicherung von unnatürlichen Isomeren.

Es wurde nun überraschend gefunden, dass sich aromatische Sulfonsäuresalze (Additionssalze) der Tetrahydrofolsäureester hervorragend zur Herstellung von optisch reinen Diastereomeren der Tetrahydrofolsäure eignen, da nur die Additionssalze des (6S,αS)- oder (6S,αR)-Diastereomeren aus organischen Lösungsmitteln auskristallisieren. Ausgehend von einem 70:30 Isomerengemisch wird schon bei einer ersten Kristallisation eine ungewöhnlich hohe Anreicherung des (6S,αS)- beziehungsweise (6S,αR)-Diastereomeren oder deren Mischungen im Kristallisat, und des (6R,αS)- beziehungsweise (6R,αR)-Diastereomeren oder deren Mischungen in der Mutterlauge erzielt, die über 99% liegen kann. Mit einer weiteren Kristallisation kann man dann meistens die optisch reinen Diastereomeren erhalten.

Gegenstand der Erfindung ist ein Verfahren zur Herstellung und Anreicherung von (6S,αS)- oder (6S,αR)-Tetrahydrofolsäureestersalzen und -Tetrahydrofolsäure, das dadurch gekennzeichnet ist, dass man in organischen Lösungsmitteln äquimolare oder angereicherte Mischungen von Diastereomeren von Additionssalze von Tetrahydrofolsäureestern mit aromatischen Sulfonsäuren herstellt oder löst, anschliessend wenigstens einmal kristallisiert, und dann das Kristallisat gegebenenfalls zu (6S,αS)- oder (6S,αR)-Tetrahydrofolsäure hydrolysiert, diese als freie Säure kristallisiert oder in Form eines Salzes isoliert.

Wenigstens einmal Kristallisieren bedeutet im Rahmen der Erfindung eine fraktionierte Kristallisation bis zur gewünschten Reinheit. Die Anzahl der Kristallisationsschritte richtet sich hierbei hauptsächlich nach dem Gehalt des oder der gewünschten Diastereomeren im Ausgangsprodukt.

Die Additionssalze der Tetrahydrofolsäureester können der Formel III entsprechen und umfassen die (6S,αS)-, (6S,αR)-, (6R,αS)- und (6R,αR)-Diastereomeren, 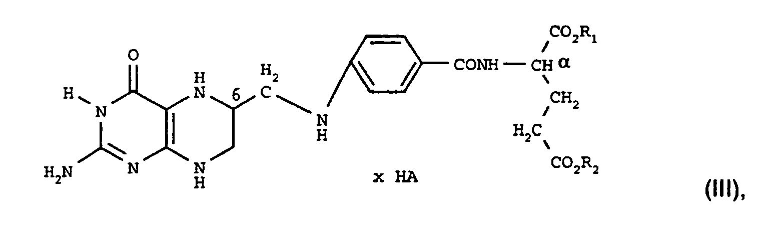 worin R₁ oder R₂ H sind, und eines von R₁ oder R₂, oder beide R₁ und R₂ unabhängig voneinander einen monovalenten Kohlenwasserstoffrest oder einen über ein C-Atom gebundenen Heterokohlenwasserstoffrest mit Heteroatomen ausgewählt aus der Gruppe -O-, -S- und -N- darstellen,
HA für eine aromatische Sulfonsäure steht,
und x eine ganze Zahl von 1 bis 6 oder eine gebrochene Zahl zwischen 0 bis 6 bedeutet.

R₁ und R₂ können unabhängig voneinander gewählt werden, bevorzugt sind sie aber identisch. Bevorzugt stellen R₁ und R₂ einen Kohlenwasserstoffrest dar. Bei R₁ und R₂ als Kohlenwasserstoffrest kann es sich um aliphatische Reste mit 1 bis 20, bevorzugt 1 bis 12, besonders bevorzugt 1 bis 8, und insbesondere bevorzugt 1 bis 4 C-Atome, um cycloaliphatische oder cycloaliphatisch-aliphatische Reste mit 3 bis 8 Ringkohlenstoffatomen und 1 bis 6 C-Atomen im aliphatischen Rest, um aromatische Kohlenwasserstoffreste mit 6 bis 14 C-Atomen, besonders bevorzugt 6 bis 10 C-Atomen, oder um aromatisch-aliphatische Reste mit 7 bis 15 C-Atomen, besonders bevorzugt 7 bis 10 C-Atomen handeln.

Bei dem Heterokohlenwasserstoffrest kann es sich um Heteroalkyl mit 2 bis 16 C-Atomen, bevorzugt 2 bis 10 C-Atomen, und besonders bevorzugt 2 bis 6 C-Atomen, um heterocycloaliphatische Reste mit 3 bis 8, bevorzugt 5 oder 6 Ringgliedern, um heterocycloaliphatisch-aliphatische Reste mit 3 bis 8, bevorzugt 5 oder 6 Ringgliedern, und 1 bis 6, bevorzugt 1 bis 4 C-Atomen im aliphatischen Rest, um heteroaromatische Reste mit bevorzugt 4 bis 13 C-Atomen, und besonders bevorzugt 4 bis 9 C-Atomen und wenigstens einem Heteroatom, und um heteroaromatisch-aliphatische Reste mit bevorzugt 4 bis 13 C-Atomen, und besonders bevorzugt 4 bis 9 C-Atomen und wenigstens einem Heteroatom, und 1 bis 6, bevorzugt 1 bis 4 C-Atomen im aliphatischen Rest handeln, wobei die Heteroreste wenigstens ein Heteroatom ausgewählt aus der Gruppe -O-, -S- und -N- und bevorzugt -O- und -N- enthalten.

Die Kohlenwasserstoffreste können zum Beispiel ausgewählt sein aus der Gruppe lineares und verzweigtes C₁-C₂₀-Alkyl, C₃-C₈- und bevorzugt C₄-C₇-Cycloalkyl, C₃-C₈-Cycloalkyl-C₁-C₆-Alkyl und bevorzugt C₄-C₇-Cycloalkyl-C₁-C₄-Alkyl, C₆-C₁₀-Aryl oder C₇-C₁₂-Aralkyl.

Die Heterokohlenwasserstoffreste können zum Beispiel ausgewählt sein aus der Gruppe C₂-C₁₆-Heteroalkyl, C₂-C₇- und bevorzugt C₄-C₅-Heterocycloalkyl, C₄-C₇-und bevorzugt C₄-C₅-Heterocycloalkyl-C₁-C₆-Alkyl , C₄-C₉- und bevorzugt C₄-C₅-Heteroaryl, und C₅-C₁₂- und bevorzugt C₅-C₁₀-Heteroaralkyl, wobei die Heteroreste 1 bis 3 und bevorzugt 1 oder 2 Heteroatome aus der Gruppe -O- und -N-enthalten.

R₁ und R₂ können lineares oder verzweigtes Alkyl sein, das bevorzugt 1 bis 12, bevorzugter 1 bis 8, und besonders bevorzugt 1 bis 4 C-Atome enthält. Beispiele sind Methyl, Ethyl, und die Isomeren von Propyl, Butyl, Pentyl, Hexyl, Heptyl, Octyl, Decyl, Dodecyl, Tetradecyl, Hexadecyl, Octadecyl und Eicosyl. Bevorzugt ist das Alkyl linear und bevorzugt ist das Alkyl Methyl, Ethyl, n-Propyl und n-Butyl. Ganz besonders bevorzugt steht Alkyl für Methyl.

R₁ und R₂ enthalten als Cycloalkyl bevorzugt 4 bis 7 und besonders bevorzugt 5 oder 6 Ringkohlenstoffatome. Beispiele für Cycloalkyl sind Cyclopropyl, Cyclobutyl, Cyclopentyl, Cyclohexyl, Cycloheptyl und Cyclooctyl. Besonders bevorzugt ist Cyclohexyl.

R₁ und R₂ enthalten als Cycloalkyl-alkyl bevorzugt 4 bis 7 und besonders bevorzugt 5 oder 6 Ringkohlenstoffatome, und bevorzugt 1 bis 4 und besonders bevorzugt 1 oder 2 C-Atome im aliphatischen Rest. Beispiele für Cycloalkyl-alkyl sind Cyclopropylmethyl oder -ethyl, Cyclobutylmethyl oder -propyl, Cyclopentylmethyl oder -ethyl, Cyclohexylmethyl oder -ethyl, Cycloheptylmethyl und Cyclooctylmethyl. Besonders bevorzugt ist Cyclohexylmethyl oder -ethyl.

R₁ und R₂ können als Aryl für Naphthyl und bevorzugt für Phenyl stehen. R₁ und R₂ sind als Aralkyl bevorzugt Phenylalkyl mit bevorzugt 1 bis 4 C-Atomen im Alkyl. Beispiele sind Benzyl und β-Phenylethyl.

R₁ und R₂ können als Heteroalkyl zum Beispiel C₁-C₄-Alkyl-X₁-C₂-C₄-alkyl sein, worin X₁ für O oder NC₁-C₄-Alkyl steht. Beispiele sind Methoxyethyl und Ethoxyethyl.

R₁ und R₂ können als Heterocycloalkyl zum Beispiel Pyrrolidinyl, Piperidinyl, Morpholinyl, Tetrahydropyranyl oder Piperazinyl sein.

R₁ und R₂ können als Heterocycloalkyl-alkyl zum Beispiel Pyrrolidinylmethyl oder - ethyl, Piperidinylmethyl oder -ethyl, Morpholinylmethyl oder -ethyl, Tetrahydropyranylmethyl oder -ethyl oder Piperazinylmethyl oder -ethyl sein.

R₁ und R₂ können als Heteroaryl zum Beispiel Thiophenyl, Furanyl, Pyranyl, Pyrrolyl, Imidazolyl, Pyridinyl, Pyrimidinyl, Pyrazinyl, Indolyl, Chinolinyl, Oxazolyl oder Isooxazolyl sein.

R₁ und R₂ können als Heteroaralkyl zum Beispiel Furanylmethyl oder -ethyl, Pyranylmethyl oder -ethyl, Pyrrolylmethyl oder -ethyl, Imidazolylmethyl oder -ethyl, Pyridinylmethyl oder -ethyl, Pyrimidinylmethyl oder -ethyl, Pyrazinylmethyl oder -ethyl, Indolylmethyl oder -ethyl, Chinolinylmethyl oder -ethyl sein.

Eine bevorzugte Gruppe von Verbindungen der Formel III sind solche, worin R₁ und R₂ unabhängig voneinander C₁-C₄-Alkyl, C₅- oder C₆-Cycloalkyl, Phenyl, C₁-C₄-Alkylphenyl, Benzyl oder C₁-C₄-Alkylbenzyl darstellen. Bevorzugt sind R₁ und R₂ gleiche Reste. Ganz besonders bevorzugt stellen R₁ und R₂ C₁-C₄-Alkyl dar, zum Beispiel Methyl oder Ethyl.

In Formel III bedeutet x bevorzugt eine ganze oder gebrochene Zahl von 0.5 bis 4, besonders bevorzugt eine ganze oder gebrochene Zahl von 0.5 bis 3, und ganz besonders bevorzugt eine ganze oder gebrochene Zahl von 0.5 bis 2.

Die aromatischen Sulfonsäuren können eine bis drei, bevorzugt ein oder zwei, und besonders bevorzugt eine Sulfonsäuregruppe enthalten. Bevorzugt sind Sulfonsäuren von Kohlenwasserstoffaromaten. Die aromatischen Sulfonsäuren können unsubstituiert oder mit Halogen, linearem oder verzweigten C₁-C₈-Alkyl, bevorzugt C₁-C₄-Alkyl, linearem oder verzweigten C₁-C₈-Alkoxy, bevorzugt C₁-C₄-Alkoxy, und linearem oder verzweigten C₁-C₈-Halogenalkyl, bevorzugt C₁-C₄-Halogenalkyl substituiert sein. Einige Beispiele für Substituenten sind Methyl, Ethyl, Propyl, Butyl, Methoxy, Ethoxy, Trifluor- oder Trichlormethyl, Fluor und Chlor. Bevorzugt enthält der aromatische Rest einen Substituenten. Unter den aromatischen Gruppen sind Phenyl und Naphthyl bevorzugt.

Die aromatischen Sulfonsäuren entsprechen besonders bevorzugt der Formel IV,

R₃-SO₃H (IV),

worin R₃ unsubstituiertes oder mit F, Cl, Br, C₁-C₄-Alkyl, C₁-C₄-Halogenalkyl oder C₁-C₄-Alkoxy substituiertes Phenyl darstellt. Einige spezifische Beispiele für R₃ sind Phenyl, Methylphenyl, Fluorphenyl, Chlorphenyl, Trichlormethylphenyl und Trifluormethylphenyl.

Besonders bevorzugte Verbindungen der Formel III sind solche, worin R₁ und R₂ je Methyl darstellen, x für 1 oder 2 oder für eine gebrochene Zahl zwischen 0.5 und 2 steht, und HA Phenyl-, Toluyl-, Fluor-, Chlor- oder Trifluormethylphenyl-sulfonsäure bedeutet. Substituierte Reste sind bevorzugt p-Toluyl-, p-Fluor-, p-Chlor- oder p-Trifluormethylphenyl.

Ganz besonders bevorzugte Verbindungen der Formel III sind solche, worin R₁ und R₂ je Methyl darstellen, x für 1 oder 2 oder für eine gebrochene Zahl zwischen 0.5 und 2 steht, und HA Phenyl- oder p-Toluylsulfonsäure bedeutet.

Die erfindungsgemäss verwendeten Additionssalze der Tetrahydrofolsäureester sind neu und können zum Beispiel durch Veresterung von Tetrahydrofolsäure in Gegenwart von Sulfonsäuren, oder durch Veresterung von Tetrahydrofolsäuresalzen in einem polaren organischen Lösungsmittel hergestellt werden.

Man kann auch von Folsäure ausgehen, und diese in an sich bekannter Weise in Gegenwart von heterogenen oder homogenen Hydrierkatalysatoren mit Wasserstoff hydrieren. Die Hydrierung kann auch diastereoselektiv geführt werden, wenn man in einem polaren Reaktionsmedium, zum Beispiel einem wässrigen oder alkoholischen Reaktionsmedium, in Gegenwart von im Reaktionsmedium löslichen chiralen Hydrierkatalysatoren mit Wasserstoff hydriert. Geeignete Hydrierkatalysatoren sind bekannt. Es handelt sich insbesondere um Metallkomplexe von Rh, Ir oder Ru mit ditertiären Diphosphinen, wie sie zum Beispiel von H. Brunner und W. Zettlmeier, Handbook of Enantioselective Catalysis, Vol. II: Ligand References, VCH Verlagsgesellschaft mbH Weinheim (1993), beschrieben werden. Die erhaltene Tetrahydrofolsäure kann in an sich bekannter Weise anschliessend verestert werden. Wenn die Hydrierung in einem Alkohol als Lösungsmittel und in Gegenwart einer Sulfonsäure unter Reaktionsbedingungen erfolgt, die zu einer Veresterung der Folsäure führen, gelangt man direkt zu den Additionssalzen aus den entsprechenden Tetrahydrofolsäureestern und Sulfonsäuren.

Man kann aber auch von Folsäureestern ausgehen, und diese in an sich bekannter Weise in Gegenwart von heterogenen oder homogenen Hydrierkatalysatoren mit Wasserstoff hydrieren. Die Hydrierung kann auch diastereoselektiv geführt werden, wenn man in einem polaren Reaktionsmedium, zum Beispiel einem alkoholischen Reaktionsmedium, in Gegenwart von im Reaktionsmedium löslichen chiralen Hydrierkatalysatoren mit Wasserstoff hydriert. Die erhaltenen Tetrahydrofolsäureester können anschliessend mit Sulfonsäuren in Additionssalze übergeführt werden. Die Hydrierung kann wie zuvor beschrieben mit in Alkoholen löslichen Metallkomplexen aus Ir, Rh oder Ru und ditertiären Diphosphinen als Hydrierkatalysatoren durchgeführt werden. Wenn die Hydrierung in einem Alkohol als Lösungsmittel und in Gegenwart einer Sulfonsäure durchgeführt wird, gelangt man direkt zu den Additionssalzen aus den entsprechenden Tetrahydrofolsäureestern und Sulfonsäuren. Verwendet man für die Hydrierung Additionssalze aus Folsäureestern mit Sulfonsäuren, so gelangt man ebenfalls direkt zu den Additionssalzen von Tetrahydrofolsäureestern und Sulfonsäuren.

Unter äquimolaren oder angereicherten Mischungen werden im Rahmen der Erfindung Mischungen verstanden, die entweder gleiche Mengen von Diastereomeren mit (6S)- und (6R)-Konfiguration oder einen Überschuss eines Diastereomeren mit (6S)- oder (6R)-Konfiguration enthalten. Man kann auch Mischungen von Diastereomeren (6S)- und (6R)-Konfiguration einsetzen, die entweder (αS)- oder (αR)-Konfiguration aufweisen, oder Mischungen von Diasteromerenpaaren mit (6S)- und (6R)-Konfiguration und unterschiedlicher Konfiguration am α-C-Atom. Die Mischungen können das (6S,αS)- beziehungsweise (6S,αR)-Diastereomere in einem Anteil von wenigstens 5, bevorzugt wenigstens 20, und besonders bevorzugt wenigstens 30 Prozent und bis zu etwa 75 Prozent oder mehr enthalten.

Geeignete organische Lösungsmittel sind polare organische Lösungsmittel, die bevorzugt wenigstens 1 g Additionssalz eines Teterahydrofolsäureesters pro Liter Lösungsmittel bei Siedetemperatur zu lösen vermögen. Beispiele für Lösungsmittel sind Halogenkohlenwasserstoff (Methylenchlorid, Chloroform, Tetrachlorethan, Chlorbenzol); Ether (Diethylether, Dibutylether, Tetrahydrofuran, Dioxan, Ethylenglykoldimethyl- oder -diethylether); Carbonsäureester und Lactone (Essigsäuremethylester, Essigsäureethylester, Propionsäuremethylester, Valerolacton); N,N-substituierte Carbonsäureamide und Lactame (Dimethylformamid, Dimethylacetamid, N-Methylpyrrolidon); Ketone (Aceton, Methylisobutylketon, Cyclohexanon); Sulfoxide und Sulfone (Dimethylsulfoxid, Dimethylsulfon, Tetramethylensulfon); und Alkohole (Methanol, Ethanol, n- oder i-Propanol, n-, i- oder t-Butanol, Pentanol, Hexanol, Cyclohexanol, Cyclohexandiol, Hydroxymethyl- oder Dihydroxymethylcyclohexan, Benzylalkohol, Ethylenglykol, Diethylenglykol, Propandiol, Butandiol, Ethylenglykolmonomethyl- oder -monoethylether, und Diethylenglykolmonomethyl- oder -monoethylether. Bevorzugt sind Ethanol und besonders Methanol. Es können auch Gemische von wenigstens zwei Lösungsmitteln verwendet werden.

Besonders bevorzugt werden Alkohole oder Mischungen aus Alkoholen mit wenigstens einem weiteren Lösungsmittel verwendet. Der Anteil an einem Alkohol beträgt bevorzugt wenigstens 30, besonders bevorzugt wenigstens 50 und insbesondere wenigstens 70 Volumenprozent. Ganz besonders bevorzugt wird nur Alkohol, zum Beispiel Methanol, oder Mischungen von Alkohol mit alkoholmischbaren Lösungsmitteln, zum Beispiel Methanol mit Ethern verwendet.

Im Einzelnen kann das Verfahren zum Beispiel so durchgeführt werden, dass man äquimolare oder angereicherte Mischungen von Diastereomeren aus Additionssalzen von Tetrahydrofolsäureestern mit aromatischen Sulfonsäuren mit einem Lösungsmittel mischt und anschliessend das Gemisch zur Lösung der Additionssalze von Tetrahydrofolsäureestern und aromatischen Sulfonsäuren erwärmt. Die Erwärmung kann bis zur Siedetemperatur des Lösungsmittels vorgenommen werden. Danach kühlt man die Lösung bis höchstens zum Festpunkt eines Lösungsmittels ab, wobei das (6S,αS)- oder (6S,αR)-Diastereomere oder beide Diastereomeren entweder spontan, oder durch Animpfen mit dem oder den gewünschten Diastereomeren, oder durch Einengen der Lösung auskristallisieren, und dann in üblicher Weise mittels Filtration abgetrennt werden können.

Es hat sich als besonderer Vorteil erwiesen, dass man zur Herstellung oder Anreicherung der Additionssalze von Tetrahydrofolsäureestern mit aromatischen Sulfonsäuren auch direkt die Reaktionslösungen der Hydrierung von Folsäureestern, oder der Hydrierung von Additionssalzen von Folsäureestern und aromatischen Sulfonsäuren verwenden kann.

Ausgehend von einem 70:30 Isomerengemisch wird bei der ersten Kristallisation bereits eine ausserordentlich hohe Anreicherung beobachtet, die zum Beispiel völlig überraschend bis zu über 99% betragen kann. Um die reinen (6S,αS)- oder (6S,αR)-Diastereomeren herzustellen, bedarf es in der Folge nur noch weniger, zum Beispiel bis zu drei, überraschend oft nur noch eines einzigen Kristallisationsschrittes.

Die beobachtete Anreicherung der (6S,αS)- beziehungsweise (6S,αR)-Diastereomeren im Kristallisat ist so hoch und die Kristallisationsfähigkeit dieser Isomeren so ausgezeichnet, dass man das erfindungsgemässe Verfahren sogar zur Isolierung von (6S,αS)- oder (6S,αR)-Diastereomeren aus Mutterlaugen einsetzen kann, die überwiegend (6R,αS)- oder (6R,αR)-Diastereomere enthalten. Die erfindungsgemässe Methode eignet sich hervorragend für Trennverfahren im industriellen Massstab.

Die nach der Trennung erhaltenen Additionssalze von (6S,αS)- oder (6S,αR)-Tetrahydrofolsäureester mit Sulfonsäuren können anschliessend in an sich bekannter Weise hydrolysiert werden, zum Beispiel mit Basen wie NaOH oder KOH. Man erhält demnach die entsprechenden (6S,αS)- oder (6S,αR)-Tetrahydrofolsäuren. Diese Tetrahydrofolsäuren können in stabiler Form als freie Säuren durch Kristallisation isoliert werden, wie es zum Beispiel in der EP-A-0 682 026 beschrieben ist. Durch Zugabe von Säuren, zum Beispiel Sulfonsäuren, können die Salze der Tetrahydrofolsäuren ebenfalls kristallisiert und gewünschtenfalls weiter angereichert werden (EP-0 495 204).

Die folgenden Beispiele können mit ähnlichem Erfolg durchgeführt werden durch Ersetzen der generisch oder spezifisch beschriebenen Reaktanden und/oder Verfahrensbedingungen dieser Erfindung durch solche die in den folgenden Beispielen aufgeführt sind. Ebenso sind die folgenden spezifischen Ausführungsformen rein beispielhaft und in keiner Art und Weise limitierend auf den Rest der Offenbarung zu sehen.
Die gesamte Offenbarung aller Anmeldungen, Patente und Publikationen, die in diesem Text zitiert sind, sind durch Referenz miteingeschlossen.
Auf Basis der vorangehenden Beschreibung kann ein Fachmann auf dem Gebiet ohne Weiteres die entscheidenden Elemente der Erfindung entnehmen und ohne vom Grundgedanken und vom Umfang der Erfindung abzuweichen, Änderungen und Ergänzungen anbringen und dadurch die Erfindung an unterschiedliche Bedürfnisse und Bedingungen anpassen.

Die folgenden Abkürzungen werden verwendet: 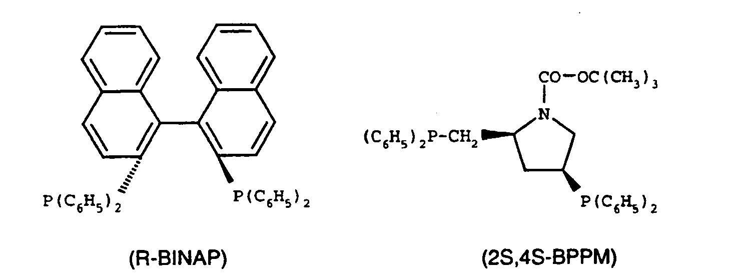 und COD steht für Cyclooctadien.

Die optische Ausbeute beziehungsweise das Verhältnis von (6S,αS)- zum (6R,αS)-Diastereomeren bzw. des (6S,αR)- zum (6R,αR)-Diastereomeren wird folgendermassen mittels Hochdruckflüssigkeitschromatographie direkt im Kristallisat oder in der Mutterlauge bestimmt:
0.5 mg des Kristallisates oder 15 mg der Mutterlauge werden in 1 ml Lösungsmittel gelöst, welches aus 6.8 g β-Cyclodextrin und 270 ml 37% Formaldehyd in 1000 ml Wasser hergestellt wird. Die Trennung erfolgt mittels einer Säule Nucleosil C-8, 5 mm, 240 x 4 mm der Firma Macherey-Nagel und einem Fliessmittel, das folgendermassen hergestellt wird: 6.8 g β-Cyclodextrin werden in einer Mischung aus 8.5 ml Triethylamin, 850 ml Wasser und 150 ml Acetonitril gelöst. Der pH-Wert der Lösung wird durch Zugabe von Essigsäure auf pH = 7.5 eingestellt, und es werden noch 270 ml 37% Formaldehyd zugegeben. Die Detektion der Diastereomeren erfolgt bei einer Wellenlänge von 300 nm.

Die Herstellung und Anreicherung sowie der Transfer von Lösungen und Suspensionen erfolgt unter Ausschluss von Sauerstoff unter Verwendung von Schutzgasen wie zum Beispiel Stickstoff oder Edelgasen.

### Beispiele

### A Herstellung von Lösungen von Additionssalzen aus Tetrahydrofolsäureestern und Sulfonsäuren

### Beispiel A1

### a Herstellung von (αS)-Folsäuredimethylester-benzolsulfonat

800 g (αS)-Folsäure-dihydrat (1.68 mmol) werden bei 40°C in eine Lösung aus 530 g Benzolsulfonsäure (3.35 mmol) und 20 Litern wasserfreiem Methanol unter einer Stickstoffatmosphäre eingetragen. Das Gemisch wird eine halbe Stunde unter Rückfluss erhitzt, abgekühlt und auf ein Volumen von 5 Litern eingeengt. Das abgeschiedene Produkt wird abgenutscht, mit 1 Liter Methanol gewaschen und im Trockenschrank bei 40°C und 20 mbar getrocknet. Man erhält 966 g (αS)-Folsäuredimethylester-benzolsulfonat (1.45 mmol, 86% der theoretischen Ausbeute) Das Produkt enthält 26.2% Benzolsulfonsäure, 1.67% Wasser und 2.26% Methanol.
Die Substanz zersetzt sich oberhalb von 150°C.
¹H-NMR in DMSO-d6: 8.78 (1 H, s), 8.46 (2H, bs), 8.32 (1H, d), 7.64-7.68 (m), 7.35-7.40 (m), 6.66 (2H, d), 0.8 (2H, s), 4.39 (1 H, m), 3.62 (3H, s), 3.57 (3H, s), 2.42 (2H, m), 1.98-2.11 (2H, m).

### b Herstellung einer Lösung eines (6S,αS)/(6R,αS)-Diastereomerengemisches von Tetrahydrofolsäuredimethylester-benzolsulfonat durch Hydrierung von (αS)-Folsäuredimethylester-benzolsulfonat

6.72 mg [Ir(COD)Cl]2 (10 µmol) und 15.57 mg (25 µmol) R-BINAP werden eingewogen, entgast und in Dichlormethan gelöst. Dichlormethan wird im Hochvakuum abkondensiert und der Rückstand in 5 ml Methanol aufgenommen. 1.25 g (αS)-Folsäuredimethylester-benzolsulfonat gemäss Beispiel A1a (2 mmol) werden in 25 ml Methanol suspendiert und zum Katalysator gegeben. Die Suspension wird im Stickstoffgegenstrom in einen 100 ml Autoklaven gegeben und so lange hydriert, bis keine Wasserstoffaufnahme mehr erfolgt. COD steht für Cyclooctadien. Man erhält Tetrahydrofolsäuredimethylester-benzolsulfonat. Das Verhältnis der Diastereomeren (6S,αS):(6R,αS) beträgt 74:26.

### c Herstellung einer Lösung eines (6S,αS)/(6R,αS)-Diastereomerengemisches von Tetrahydrofolsäuredimethylester-benzolsulfonat mit einem Überschuss des (6R,αS)-Diastereomers durch Hydrierung von (αS)-Folsäuredimethylester-benzolsulfonat

6.72 mg [Ir(COD)Cl]2 (10 µmol) und 13.84 mg (25 µmol) (2S,4S)-BPPM werden eingewogen, entgast und in Dichlormethan gelöst. Dichlormethan wird im Hochvakuum abkondensiert und der Rückstand in 5 ml Methanol aufgenommen. 1.25 g (αS)-Folsäuredimethylester-benzolsulfonat gemäss Beispiel A1a (2 mmol) werden in 25 ml Methanol suspendiert und zum Katalysator gegeben. Die Suspension wird im Stickstoffgegenstrom in einen 100 ml Autoklaven gegeben und während 17 Stunden hydriert. Man erhält Tetrahydrofolsäuredimethylester-benzolsulfonat. Das Verhältnis der Diastereomeren (6S,αS):(6R,αS) beträgt 34:66.

### Beispiel A2

### Herstellung einer Lösung eines äquimolaren (6S,αS)/(6R,αS)-Diastereomerengemisches von Tetrahydrofolsäuredimethylester-benzolsulfonat durch Veresterung von Tetrahydrofolsäure

20 g einer äquimolaren Mischung von (6S,αS)- und (6R,αS)-Tetrahydrofolsäure (44.9 mmol) werden in 900 ml Methanol mit 10.65 g Benzolsulfonsäure (67.35 mmol) versetzt und 7 Stunden unter Rückfluss erhitzt. Es entsteht eine Lösung von (6S,αS)- und (6R,αS)-Tetrahydrofolsäuredimethylester-benzolsulfonat.

### Beispiel A3

### Herstellung einer Lösung eines (6S,αS)/(6R,αS)-Diastereomerengemisches von 70:30 von Tetrahydrofolsäuredimethylester-benzolsulfonat durch Veresterung von Tetrahydrofolsäure mit einem (6S,αS)/(6R,αS)-Diastereomerenverhältnis von 70:30

5.31 g Tetrahydrofolsäure (11.92 mmol) mit einem Diastereomerenverhältnis von (6S,αS):(6R,αS) = 70:30 (hergestellt nach EP 0 495 204 B1) werden in 230 ml Methanol mit 2.83 g Benzolsulfonsäure (17.88 mmol) 7 Stunden unter Rückfluss erhitzt. Man erhält eine Lösung von Tetrahydrofolsäuredimethylester-benzolsulfonat mit einem Diastereomerenverhältnis von (6S,αS):(6R,αS) = 70:30.

### Beispiel A4

### Herstellung einer äquimolaren Lösung der Diastereomeren von (6S,αS)- und (6R,αS)-Tetrahydrofolsäuredimethylester-toluolsulfonat

10 g einer äquimolaren Mischung von (6S,αS)- und (6R,αS)-Tetrahydrofolsäure (22.45 mmol) werden in 450 ml Methanol mit 6.41 g Toluolsulfonsäure-monohydrat (33.67 mmol) versetzt und 7 Stunden unter Rückfluss erhitzt. Man erhält eine Lösung von Tetrahydrofolsäuredimethylester-toluolsulfonat mit einem Diastereomerenverhältnis von (6S,αS):(6R,αS) = 1:1.

### Beispiel A5

### Herstellung einer äquimolaren Lösung von Diastereomeren des (6S,αS)- und (6R,αS)-Tetrahydrofolsäuredimethylester-naphthalin-1-sulfonates

3 g einer äquimolaren Mischung von (6S,αS)- und (6R,αS)-Tetrahydrofolsäure (6.73 mmol) werden in 130 ml Methanol mit 2.33 g Naphthalin-1-sulfonsäurenatriumsalz (10.1 mmol) und 4.7 ml 2 M HCl versetzt und 7 Stunden unter Rückfluss erhitzt. Man erhält eine Lösung von Tetrahydrofolsäuredimethylester-naphthalin-1-sulfonat mit einem Diastereomerenverhältnis von (6S,αS):(6R,αS) = 1:1.

### B Isolierungs- und Anreicherungsverfahren

### Beispiel B1

### Isolierung und Anreicherung von (6S,αS)-Tetrahydrofolsäuredimethylesterbenzolsulfonat

a Die gemäss Beispiel A1b erhaltene Lösung von Tetrahydrofolsäuredimethylester-benzolsulfonat mit einem Diastereomerenanteil des (6S,αS)-Diastereomeren von 74% wird unter Ausschluss von Sauerstoff auf 1/6 des Volumens eingeengt. Die so erhaltene Suspension wird unter Stickstoffatmosphäre für 2 Stunden bei 4°C gelagert, das abgeschiedene Produkt wird abgesaugt, mit wenig kaltem Methanol gewaschen und bei 40°C und 20 mbar getrocknet. Man erhält 0.55 g Tetrahydrofolsäuredimethylester-benzolsulfonat (0.87 mmol, 44% der theoretischen Ausbeute). Das Verhältnis der Diastereomeren des Tetrahydrofolsäuredimethylester-benzolsulfonates (6S,αS):(6R,αS) beträgt 99:1. [a]₅₈₉= -69.8° (c = 1 in Dimethylsulfoxid).
   Die Substanz zersetzt sich oberhalb von 150°C.
   1^{H}-NMR in DMSO-d6: 10.61 (1 H, bs), 8.35 (1H, d), 7.6-7.74 (m), 7.51 (1H, bs), 7.30-7.37 (m), 6.70 (2H, d, 2H, bs), 4.42 (2H, m), 3.63 (3H, s), 3.58 (3H, s), 3.50 (1 H, m), 3.38 (1 H, m), 3.28 (1 H, m), 2.44 (2H, m), 2.01-2.13 (2H, m)
**b Isolierung und Anreicherung von (6S,αS)-Tetrahydrofolsäuredimethylester-benzolsulfonat aus der Lösung gemäss Beispiel A1c**
   Die gemäss Beispiel A1c erhaltene Lösung von Tetrahydrofolsäuredimethylester-benzolsulfonat mit einem Anteil des (6S,αS)-Diastereomeren von 34% wird unter Ausschluss von Sauerstoff unter Stickstoffatmosphäre für 2 Stunden bei 4°C gelagert. Danach wird das abgeschiedene Produkt abgesaugt, mit wenig kaltem Methanol gewaschen und dann bei 40°C und 20 mbar getrocknet. Man erhält 0.2 g Tetrahydrofolsäuredimethylester-benzolsulfonat mit einem Anteil des (6S,αS)-Diastereomeren von 96.6%.
**c Isolierung und Anreicherung von (6S,αS)-Tetrahydrofolsäuredimethylester-benzolsulfonat aus der Lösung gemäss Beispiel A2**
   Die klare Lösung aus Beispiel A2 wird auf Raumtemperatur abgekühlt und über Nacht gerührt. Der abgeschiedene Feststoff wird abgesaugt, mit Methanol und tert.-Butylmethylether gewaschen und bei 30°C und 10 mbar getrocknet. Man erhält 9.62 g farbloses kristallines Tetrahydrofolsäuredimethylester-benzolsulfonat (15.24 mmol) mit einem Anteil des (6S,αS)-Diastereomeren von 99.1 %. (Aus der Mutterlauge B1c kann das (6R,αS)-Tetrahydrofolsäuredimethylester-benzolsulfonat wie unter Beispiel B5 beschrieben hergestellt werden.)
   4 g (6.34 mmol) des erhaltenen Tetrahydrofolsäuredimethylester-benzolsulfonates mit einem Anteil des (6S,αS)-Diastereomeren von 99.1% werden in 220 ml siedendem Methanol gelöst. Man lässt auf Raumtemperatur abkühlen, lässt über Nacht stehen und saugt den abgeschiedenen Feststoff ab. Man wäscht mit Methanol und tert.-Butylmethylether und trocknet bei 35°C und 10 mbar. Man erhält 3.08 g (4.88 mmol) farbloses kristallines Tetrahydrofolsäuredimethylester-benzolsulfonat mit einem Anteil des (6S,αS)-Diastereomeren von 99.5%.
**d Isolierung und Anreicherung von (6S,αS)-Tetrahydrofolsäuredimethylester-benzolsulfonat aus der Lösung gemäss Beispiel A3**
   Man lässt die nach Beispiel A3 erhaltene Lösung auf Raumtemperatur abkühlen und impft die Lösung bei 60°C mit diastereomerenreinem (6S,αS)-Tetrahydrofolsäuredimethylester-benzolsulfonat an. Nach Stehen über Nacht saugt man den abgeschiedenen Feststoff ab, wäscht mit Methanol und tert.-Butylmethylether und trocknet bei 35°C und 10 mbar. Man erhält 3.46 g (5.48 mmol) Tetrahydrofolsäuredimethylester-benzolsulfonat mit einem Anteil des (6S,αS)-Diastereomeren von 99.9%.

### Beispiel B2

### Isolierung und Anreicherung von (6S,αS)-Tetrahydrofolsäuredimethylester-Toluolsulfonat

Die unter Beispiel A4 erhaltene äquimolare Mischung von Tetrahydrofolsäuredimethylester-toluolsulfonat wird auf Raumtemperatur abgekühlt und über Nacht gerührt. Der abgeschiedene Feststoff wird abgesaugt, mit Methanol und tert.-Butylmethylether gewaschen und bei 30°C und 10 mbar getrocknet. Man erhält 5.53 g farbloses kristallines Tetrahydrofolsäuredimethylester-toluolsulfonat (9.54 mmol) mit einem Anteil des (6S,αS)-Diastereomeren von 99.1%.
5.2 g (8.97 mmol) des so erhaltenen Tetrahydrofolsäuredimethylester-toluolsulfonates mit einem Anteil des (6S,αS)-Diastereomeren von 99.1% werden in 182 ml siedendem Methanol gelöst. Man lässt auf Raumtemperatur abkühlen, rührt drei Stunden bei Raumtemperatur und saugt den abgeschiedenen Feststoff ab. Man wäscht mit Methanol und tert.-Butylmethylether und trocknet bei 35°C und 10 mbar. Man erhält 4.43 g (7.64 mmol) farbloses kristallines Tetrahydrofolsäuredimethylester-toluolsulfonat mit einem Anteil des (6S,αS)-Diastereomeren von 99.8%.

### Beispiel B3

### Isolierung und Anreicherung von (6S,αS)-Tetrahydrofolsäuredimethylesternaphthalin-1-sulfonat

Die unter Beispiel A5 erhaltene Lösung wird auf Raumtemperatur abgekühlt und über Nacht gerührt. Der abgeschiedene Feststoff wird abgesaugt und bei 30°C und 10 mbar getrocknet. Man erhält 0.34 g farbloses Tetrahydrofolsäuredimethylester-naphthalin-1-sulfonat (0.55 mmol) mit einem Anteil des (6S,αS)-Diastereomeren von 62.7%.

### Beispiel B4

### Herstellung von (6S,αS)-Tetrahydrofolsäure-benzolsulfonat durch Hydrolyse von Tetrahydrofolsäuredimethylester-benzolsulfonat

0.55 g Tetrahydrofolsäuredimethylester-benzolsulfonat (0.95 mmol) gemäss Beispiel B1 a und 0.32 g Natriumcarbonat (3.02 mmol) werden unter Ausschluss von Sauerstoff in 4 ml Wasser gelöst. Man erhitzt auf 85°C und stellt nach 30 Minuten den pH-Wert mit 37%iger Salzsäure auf pH = 7.5 ein. Bei 75°C werden 0.2 g Benzolsulfonsäure in 0.6 ml Wasser zugegeben und anschliessend wird der pH-Wert mit 37%iger Salzsäure auf pH = 0.8 eingestellt. Man lässt die Lösung auf Raumtemperatur abkühlen und rührt noch drei Stunden. Das Produkt wird abgenutscht und im Trockenschrank bei 30°C und 20 mbar 4 Tage lang getrocknet. Man erhält 8.4 g Tetrahydrofolsäure-benzolsulfonat (13.92 mmol, 88% der theoretischen Ausbeute).

Das Diastereomerenverhältnis des Tetrahydrofolsäure-benzolsulfonates (6S,αS):(6R,αS) beträgt 99:1.
Die Eigenschaften des Tetrahydrofolsäure-benzolsulfonates sind mit denen des in EP 0495204 B1 beschriebenen Produktes identisch.

### Beispiel B5

### Isolierung von angereichertem (6R,αS)-Tetrahydrofolsäuredimethylesterbenzolsulfonat

Die Mutterlauge aus Beispiel B1c wird auf ein Viertel des Volumens eingeengt. Man kühlt auf 0°C ab, impft mit diastereomerenreinem (6S,αS)-Tetrahydrofolsäuredimethylester-benzolsulfonat an und saugt 1.5 g Tetrahydrofolsäuredimethylester-benzolsulfonat mit einem Diastereomerenverhältnis von (6S,αS):(6R,αS)= 97:3 ab. Die Mutterlauge wird bis zur Trockene eingeengt. Der ölige Rückstand wird mit 200 ml Diethylether versetzt und 2 Stunden bei 0°C gerührt. Der abgeschiedene Feststoff wird abgesaugt, mit Diethylether gewaschen und bei 30°C und 20 mbar getrocknet. Man erhält 14.8 g Tetrahydrofolsäuredimethylester-benzolsulfonat mit einem (6R,αS)/ (6S,αS)-Diastereomerenverhältnis von 80:20.

## Patentansprüche

1. Verfahren zur Herstellung und Anreicherung von (6S,αS)- oder (6S,αR)-Tetrahydrofolsäureestersalzen und -Tetrahydrofolsäure, **dadurch gekennzeichnet, dass** man in organischen Lösungsmitteln äquimolare oder angereicherte Mischungen von Diastereomeren von Additionssalzen von Tetrahydrofolsäureestern mit aromatischen Sulfonsäuren herstellt oder löst, anschliessend wenigstens einmal kristallisiert, und dann das Kristallisat gegebenenfalls zu (6S,αS)- oder (6S,αR)-Tetrahydrofolsäure hydrolysiert, diese als freie Säure kristallisiert oder in Form eines Salzes isoliert.

2. Verfahren gemäss Anspruch 1, **dadurch gekennzeichnet, dass** die Additionssalze der Tetrahydrofolsäureester der Formel III entsprechen, die die (6S,αS)-, (6S,αR)-, (6R,αS)- und (6R,αR)-Diastereomeren umfasst, 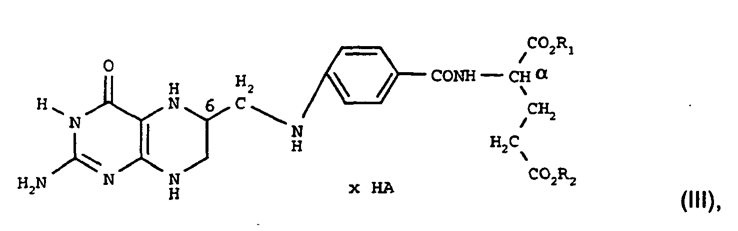 worin R₁ oder R₂ H sind, und eines von R₁ oder R₂, oder beide R₁ und R₂ unabhängig voneinander einen monovalenten Kohlenwasserstoffrest oder einen über ein C-Atom gebundenen Heterokohlenwasserstoffrest mit Heteroatomen ausgewählt aus der Gruppe -O-, -S- und -N- darstellen,
HA für eine aromatische Sulfonsäure steht,
und x eine ganze Zahl von 1 bis 6 oder eine gebrochene Zahl zwischen 0 bis 6 bedeutet

3. Verfahren gemäss Anspruch 2, **dadurch gekennzeichnet, dass** R₁ und R₂ C₁-C₄-Alkyl bedeutet.

4. Verfahren gemäss Anspruch 3, **dadurch gekennzeichnet, dass** R₁ und R₂ für Methyl stehen.

5. Verfahren gemäss Anspruch 2, **dadurch gekennzeichnet, dass** x in Formel III für die Zahlen 1 oder 2, oder eine gebrochene Zahl zwischen 0.5 und 2 steht.

6. Verfahren gemäss Anspruch 1, **dadurch gekennzeichnet, dass** die aromatischen Sulfonsäuren der Formel IV entsprechen,
R₃-SO₃H (IV),
worin R₃ unsubstituiertes oder mit C₁-C₄-Alkyl, C₁-C₄-Halogenalkyl oder C₁-C₄-Alkoxy substituiertes Phenyl darstellt.

7. Verfahren gemäss Anspruch 6, **dadurch gekennzeichnet, dass** die aromatische Sulfonsäure Benzol- oder -p-Toluolsulfonsäure ist.

8. Verfahren gemäss Anspruch 2, **dadurch gekennzeichnet, dass** in den Verbindungen der Formel III R₁ und R₂ je Methyl darstellen, x für 1 oder 2 oder für eine gebrochene Zahl zwischen 0.5 und 2 steht, und HA Phenyl-, Toluyl-, Fluor-, Chlor- oder Trifluormethylphenyl-sulfonsäure bedeutet.

9. Verfahren gemäss Anspruch 8, **dadurch gekennzeichnet, dass** in den Verbindungen der Formel III R₁ und R₂ je Methyl darstellen, x für 1 oder 2 oder für eine gebrochene Zahl zwischen 0.5 und 2 steht, und HA Phenyl- oder p-Toluylsulfonsäure bedeutet.

10. Verfahren gemäss Anspruch 1, **dadurch gekennzeichnet, dass** die Mischungen das (6S,αS)- beziehungsweise (6S,αR)-Diastereomere in einem Anteil von wenigstens 5 Gewichtsprozent oder mehr enthalten.

11. Verfahren gemäss Anspruch 1, **dadurch gekennzeichnet, dass** es sich bei den organischen Lösungsmitteln um polare organische Lösungsmittel handelt, die wenigstens 1 g Additionssalz eines Tetrahydrofolsäureesters pro Liter Lösungsmittel bei Siedetemperatur lösen.

12. Verfahren gemäss Anspruch 11, **dadurch gekennzeichnet, dass** man Alkohole oder Mischungen aus Alkoholen mit wenigstens einem weiteren Lösungsmittel verwendet.

13. Verfahren gemäss Anspruch 1, **dadurch gekennzeichnet, dass** man äquimolare oder angereicherte Mischungen von Diastereomeren von Additionssalzen aus Tetrahydrofolsäureestern mit aromatischen Sulfonsäuren in einem Lösungsmittel mischt und anschliessend das Gemisch zur Lösung der Additionssalze von Tetrahydrofolsäureestern und aromatischen Sulfonsäuren erwärmt, danach die Lösung abkühlt, wobei das (6S,αS)- oder (6S,αR)-Diastereomere auskristallisiert oder beide Diastereomeren auskristallisieren, und diese dann mittels Filtration abtrennt.

14. Verfahren gemäss Anspruch 1, **dadurch gekennzeichnet, dass** man Reaktionslösungen aus der Hydrierung von Folsäureestern, oder aus der Hydrierung von Additionssalzen von Folsäureestern und aromatischen Sulfonsäuren, oder aus der Hydrierung von Folsäure in Gegenwart von Sulfonsäuren unter veresternden Bedingungen verwendet.

15. Verfahren gemäss Anspruch 1, **dadurch gekennzeichnet, dass** man (6S,αS)- oder (6S,αR)-Tetrahydrofoläureester-Sulfonate oder deren Gemische zu (6S,αS)- oder (6S,αR)-Tetrahydrofolsäure oder deren Gemische basisch hydrolysiert.

## Claims

1. Method for making and enriching (6S,αS) or (6S,αR) tetrahydrofolic acid ester salts and tetrahydrofolic acid, **characterised in that** equimolar or enriched mixtures of diastereomers of addition salts of tetrahydrofolic acid esters and aromatic sulphonic acids are made or dissolved in organic solvents and then crystallised at least once, and if necessary the crystallisate is then hydrolysed to (6S,αS) or (6S,αR) tetrahydrofolic acid and crystallised as free acid or isolated in the form of a salt.

2. Method according to claim 1, **characterised in that** the addition salts of the tetrahydrofolic acid esters correspond to formula III, which comprises the (6S,αS), (6S,αR), (6R,αS) and (6R,αR) diastereomers. 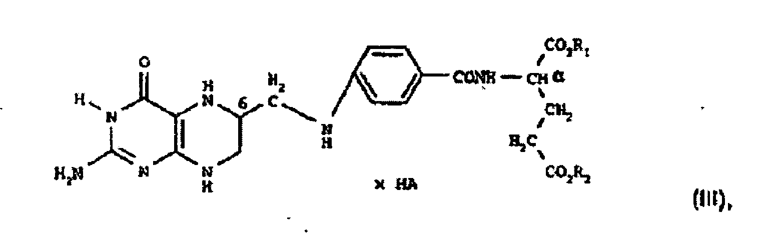 in which R₁ or R₂ are H and either of R₁ or R₂ or both R₁ and R₂ independently of each other represent a monovalent hydrocarbon residue or a C-atom bonded heterohydrocarbon residue with heteroatoms selected from the -O-, - S- and -N- group,
HA stands for an aromatic sulphonic acid,
and x means a whole number from 1 to 6 or a fractional number between 0 and 6.

3. Method according to claim 2, **characterised in that** R₁ and R₂ mean C₁-C₄-alkyl.

4. Method according to claim 3, **characterised in that** R₁ and R₂ stand for methyl.

5. Method according to claim 2, **characterised in that** x in formula III stands for the numbers 1 or 2, or a fractional number between 0.5 and 2.

6. Method according to claim 1, **characterised in that** the aromatic sulphonic acids correspond to formula IV,
R₃-SO₃H (IV),
in which R₃ represents phenyl, unsubstituted or substituted with C₁-C₄-alkyl, C₁-C₄-halogen alkyl or C₁-C₄-alkoxy.

7. Method according to claim 6, **characterised in that** the aromatic sulphonic acid is benzene or p-toluene sulphonic acid.

8. Method according to claim 2, **characterised in that** in the compounds in formula III R₁ and R₂ each represent methyl, x stands for 1 or 2 or a fractional number between 0.5 and 2 and HA means phenyl, toluyl, fluoro, chloro or trifluoro methyl phenyl sulphonic acid.

9. Method according to claim 8, **characterised in that** in the compounds in formula III R₁ and R₂ each represent methyl, x stands for 1 or 2 or a fractional number between 0.5 and 2 and HA means phenyl or p-toluyl sulphonic acid.

10. Method according to claim 1, **characterised in that** the mixtures contain the (6S,αS) or (6S,αR) diastereomers in a proportion of at least 5 percent by weight or more.

11. Method according to claim 1, **characterised in that** the organic solvents are polar organic solvents which dissolve at least 1 g addition salt of a tetrahydrofolic acid ester per litre of solvent at boiling temperature.

12. Method according to claim 11, **characterised in that** alcohols or mixtures of alcohols with at least one other solvent are used.

13. Method according to claim 1, **characterised in that** equimolar or enriched mixtures of diastereomers of addition salts of tetrahydrofolic acid esters are mixed with aromatic sulphonic acids in a solvent and the mixture is then heated to dissolve the addition salts of tetrahydrofolic acid esters and aromatic sulphonic acids, after which the solution is cooled, wherein the (6S,αS) or (6S,αR) diastereomer crystallises or both diastereomers crystallise and is or are then separated by filtration.

14. Method according to claim 1, **characterised in that** reaction solutions from the hydrogenation of folic acid esters or from the hydrogenation of addition salts of folic acid esters and aromatic sulphonic acids, or from the hydrogenation of folic acid in the presence of sulphonic acids under esterifying conditions are used.

15. Method according to claim 1, **characterised in that** (6S,αS) or (6S,αS) tetrahydrofolic acid ester sulphonates or their mixtures are alkaline hydrolysed to form (6S,αS) or (6S,αS) tetrahydrofolic acid or its mixtures.

## Revendications

1. Procédé de préparation et d'enrichissement de sels d'ester d'acide tétrahydrofolique et d'acide tétrahydrofolique (6S,αS) ou (6S,αS), **caractérisé en ce qu'**on prépare ou qu'on dissout dans des solvants organiques des mélanges équimolaires ou enrichis en diastéréomères de sels d'addition d'esters d'acide tétrahydrofolique avec des acides sulfoniques aromatiques, qu'on les cristallise ensuite au moins une fois et qu'on hydrolyse ensuite éventuellement le cristallisat en acide tétrahydrofolique (6S,αS) ou (6S,αS), qu'on cristallise ce dernier en tant qu'acide libre ou qu'on l'isole sous forme d'un sel.

2. Procédé selon la revendication 1, **caractérisé en ce que** les sels d'addition d'ester d'acide tétrahydrofolique correspondent à la formule III comprenant les diastéréomères (6S,αS), (6S,αS) et (6R,αS), (6R, αR) 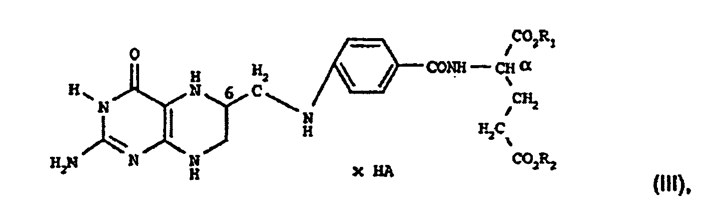 R₁ ou R₂ étant H et un élément parmi R₁ ou R₂ ou à la fois R₁ et R₂ constituant indépendamment l'un de l'autre un radical d'hydrocarbure monovalent ou un radical d'hétérohydrocarbure lié par un atome de C avec des hétéroatomes sélectionnés parmi le groupe O, S et N,
HA représentant un acide sulfonique aromatique,
et x étant un nombre entier de 1 à 6 ou un nombre fractionné allant de 0 à 6.

3. Procédé selon la revendication 2, **caractérisé en ce que** R₁ et R₂ représentent un alkyle en C₁-C₄.

4. Procédé selon la revendication 3, **caractérisé en ce que** R₁ et R₂ représentent le méthyle.

5. Procédé selon la revendication 2, **caractérisé en ce que** le x de la formule III représente les nombres 1 ou 2 ou un nombre fractionné allant de 0,5 à 2.

6. Procédé selon la revendication 1, **caractérisé en ce que** les acides sulfoniques aromatiques correspondent à la formule IV,
R₃-SO₃H (IV),
R₃ représentant un phényle non substitué ou substitué avec de l'alkyle en C₁-C₄, de l'halogénalkyle en C₁-C₄ ou de l'alcoxy en C₁-C₄.

7. Procédé selon la revendication 6, **caractérisé en ce que** l'acide sulfonique aromatique est l'acide benzène sulfonique ou l'acide p-toluènesulfonique.

8. Procédé selon la revendication 2, **caractérisé en ce que**, dans les composés de formule III, R₁ et R₂ représentent chaque fois le méthyle, x représente 1 ou 2 ou un nombre fractionné allant de 0,5 à 2 et HA le phényle-, toluylène-, fluoro-, chloro- ou trifluorométhylphényle-acide sulfonique.

9. Procédé selon la revendication 8, **caractérisé en ce que**, dans les composés de formule III, R₁ et R₂ représentent chaque fois le méthyle, x représente 1 ou 2 ou un nombre fractionné allant de 0,5 à 2 et HA le phényle- ou p-toluylène-acide sulfonique.

10. Procédé selon la revendication 1, **caractérisé en ce que** les mélanges contiennent le diastéréomère (6S,αS) ou (6S,αS) en une proportion d'au moins 5 pour cent en poids ou plus.

11. Procédé selon la revendication 1, **caractérisé en ce que** s'il s'agit, en ce qui concerne les solvants organiques, de solvants organiques polaires qui dissolvent au moins 1 g de sel d'addition d'un ester d'acide tétrahydrofolique par litre de solvant à température d'ébullition.

12. Procédé selon la revendication 11, **caractérisé en ce qu'**on utilise des alcools ou des mélanges d'alcools comprenant au moins un autre solvant.

13. Procédé selon la revendication 1, **caractérisé en ce qu'**on mélange des mélanges équimolaires ou enrichis en diastéréomères de sels d'addition d'esters d'acide tétrahydrofolique à des acides sulfoniques aromatiques dans un solvant et qu'on réchauffe ensuite le mélange pour dissoudre les sels d'addition d'esters d'acide tétrahydrofolique et les acides sulfoniques aromatiques, qu'ensuite on refroidit la solution, le diastéréomère (6S,αS) ou (6S,αS) se cristallisant ou les deux diastéréomères se cristallisant, et qu'on les sépare ensuite par filtration.

14. Procédé selon la revendication 1, **caractérisé en ce qu'**on utilise des solutions réactionnelles issues de l'hydrogénation d'esters d'acide folique ou de l'hydrogénation de sels d'addition d'esters d'acide folique et d'acides sulfoniques aromatiques ou de l'hydrogénation d'acide folique en présence d'acides sulfoniques dans des conditions d'estérification.

15. Procédé selon la revendication 1, **caractérisé en ce qu'**on hydrolyse des sulfonates d'ester d'acide tétrahydrofolique (6S,αS) ou (6S,αS) ou leurs mélanges en acide tétrahydrofolique (6S,αS) ou (6S,αS) ou leurs mélanges de manière basique.
